# EUROPEAN PATENT APPLICATION

(11) **EP 4 604 141 A1**
(43) Date of publication of application: **20.08.2025**
(21) Application number: 25156969.5
(22) Date of filing: 10.02.2025
(51) Int. Cl.: H01B 11/18, A61B 1/00, H01B 13/06, H01B 13/16, H01B 7/04

(54) **ULTRAFINE COAXIAL CABLE**

(30) Priority: 13.02.2024 US 202418440183
(71) Applicant: Creganna Unlimited Company, Ballybrit, Galway (IE)
(72) Inventor: Brown, Troy, Wilsonville, 97070-9289 (US)
(74) Representative: Ashton, Gareth Mark

(57) **Abstract**

A coaxial cable (100) includes a center conductor (110), a dielectric coating (122) applied to the center conductor forming an insulator (120) surrounding the center conductor (110), a metallization layer (132) applied to the insulator (120) forming a cable shield (130) surrounding the insulator (120), and an outer jacket (140) covering the cable shield (130), wherein the coaxial cable (100) has a wire gauge of 54 AWG or smaller. A method of manufacturing a coaxial cable (100) includes providing a center conductor (110), coating the center conductor (110) with a dielectric coating (122) to form an insulator (120) surrounding the center conductor (110), covering the insulator (120) with a metallization layer (132) to form a cable shield (130) surrounding the insulator (120), and covering the cable shield (130) with an outer jacket (140), wherein the coaxial cable (100) has a wire gauge of 54 AWG or smaller.

## Description

### BACKGROUND OF THE INVENTION

The subject matter herein relates generally to coaxial cables and methods of manufacturing coaxial cables.

Coaxial cables are used in many applications for data transmission between various electronic devices. For example, medical devices may use coaxial cables to electrically connect an instrument or tool used during a surgical procedure to a processing unit. For example, the medical devices may be used for medical imaging, such as ultrasound imaging. The medical devices may be used in endoscopy. Other medical devices, such as minimally invasive, interventional devices, may include a catheter having coaxial cable routed through lumen(s) of the catheter.

Traditional coaxial cables used in medical devices have been characterized by relatively larger diameters, limiting application in procedures requiring intricate movements within confined spaces such as intravascular or interventional procedures. These cables often pose challenges in terms of flexibility and ease of navigation, impacting the overall efficacy of some surgical techniques. There is a desire to decrease size of components of medical devices. There may also be a desire to increase the number of coaxial cables included within a medical device, requiring smaller diameter coaxial cables. Diameters of coaxial cables may be limited by factoring processes. For example, conventional coaxial cable manufacturing processes have limits to how thin the extruded walls can be made. For example, conventional extruded coaxial cables may be limited to 50 AWG (0.00099 inch (0.0250 mm)) and larger gauge wires due to extrusion limitations (for example, extrusion limits of approximately 0.0008 inch (0.020 mm) for typical extrusion materials).

### BRIEF DESCRIPTION OF THE INVENTION

In one embodiment, a coaxial cable is provided and includes a center conductor. The coaxial cable includes a dielectric coating applied to the center conductor. The dielectric coating forms an insulator surrounding the center conductor. The coaxial cable includes a metallization layer applied to the insulator. The metallization layer forms a cable shield surrounding the insulator. The coaxial cable may include an outer jacket covering the cable shield. The coaxial cable may have a wire gauge of 54 AWG (0.00062 inch (0.01575 mm)) or smaller.

In another embodiment, a method of manufacturing a coaxial cable is provided and includes providing a center conductor. The method includes coating the center conductor with a dielectric coating to form an insulator surrounding the center conductor. The method includes covering the insulator with a metallization layer to form a cable shield surrounding the insulator. The method includes covering the cable shield with an outer jacket. The coaxial cable has a wire gauge of 54 AWG (0.00062 inch (0.01575 mm)) or smaller. The coating may comprise the step of dip coating the dielectric coating.

In a further embodiment, a medical device is provided and includes an instrument which performs an operation. The medical device includes an ultrafine coaxial cable electrically connected to the instrument to transmit data between the instrument and an electrical device. The ultrafine coaxial cable including a center conductor, a dielectric coating applied to the center conductor forms an insulator surrounding the center conductor, a metallization layer applied to the insulator forms a cable shield surrounding the insulator, and an outer jacket covering the cable shield, The coaxial cable has a wire gauge of 54 AWG (0.00062 inch (0.01575 mm)) or smaller.

### BRIEF DESCRIPTION OF THE DRAWINGS

Figure 1 illustrates a coaxial cable in accordance with an exemplary embodiment which is shown electrically connecting two electrical components.
Figure 2 is an end view of the coaxial cable in accordance with an exemplary embodiment showing various layers of the coaxial cable.
Figure 3 is a cross-sectional view of the coaxial cable in accordance with an exemplary embodiment.
Figure 4 is a flow chart showing an exemplary method of manufacturing a coaxial cable.
Figure 5 is a chart showing cable outer diameter versus manufacturing process.
Figure 6 is a flow chart showing an exemplary method of manufacturing a coaxial cable.
Figure 7 is a flow chart showing an exemplary method of manufacturing a coaxial cable.
Figure 8 is a chart showing the dielectric constant and the capacitance for a 50 Ohm, 54 AWG coaxial cable using various insulator materials in accordance with an exemplary embodiment.
Figure 9 is a chart showing the dielectric constant and the outer diameter for a 50 Ohm, 54 AWG (0.00062 inch (0.01575 mm)) coaxial cable using various insulator materials in accordance with an exemplary embodiment.

### DETAILED DESCRIPTION OF THE INVENTION

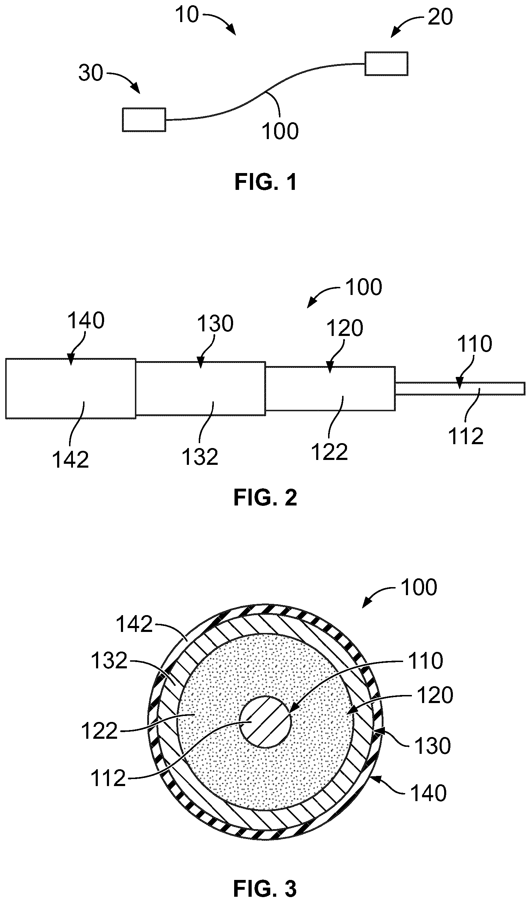

Figure 1 illustrates a coaxial cable 100 in accordance with an exemplary embodiment. Figure 2 is an end view of the coaxial cable 100 in accordance with an exemplary embodiment showing various layers of the coaxial cable 100. Figure 3 is a cross-sectional view of the coaxial cable 100 in accordance with an exemplary embodiment. The coaxial cable 100 electrically connects a first electrical component 20 and a second electrical component 30. The coaxial cable 100 is used to transmit signals (for example, data or analog signals) between the first electrical component 20 and the second electrical component 30. In an exemplary embodiment, the coaxial cable 100 is an ultrafine coaxial cable. For example, the coaxial cable 100 has a wire gauge less than 50 AWG (0.00099 inch (0.0250 mm)). In an exemplary embodiment, the coaxial cable 100 has a wire gauge of 54 AWG (0.00062 inch (0.01575 mm)) or smaller. The coaxial cable 100 may have a wire gauge of 60 AWG (0.00031 inch (0.00785 mm) in various embodiments. The coaxial cable 100 may have a wire gauge of 64 AWG in various embodiments.

In an exemplary embodiment, the coaxial cable 100 forms part of a medical device 10. For example, the medical device may be a medical device or a diagnostic device. In various embodiments, the medical device 10 is part of an ultrasound system. For example, the first electrical component 20 is an ultrasound transducer or ultrasound probe. The coaxial cable 100 electrically connects the ultrasound transducer with the second electrical component 30, which may be an ultrasound processing unit, such as a computer. In other various embodiments, the medical device 10 is part of an endoscopy system. For example, the first electrical component 20 is a camera, such as an endoscope. The coaxial cable 100 electrically connects the camera with the second electrical component 30, which may be a processing unit, such as a computer. In other various embodiments, the medical device 10 includes a catheter having a lumen and the coaxial cable 100 is routed through the lumen. Other types of medical devices 10 may utilize the coaxial cable 100 in alternative embodiments. The ultrafine coaxial cable 100 may be routed inside of the catheter or the device, such as between a sensor on one end and a connector on the other end, where the ultrafine coaxial cable 100 travels through the catheter/device making the connections therebetween. The ultrafine coaxial cable 100 is flexible and configured to be utilized in small spaces. The ultrafine coaxial cable 100 may have control the impedance and noise immunity for use in the medical device 10. The ultrafine coaxial cable 100 is configured to transmit high speed data, such as high-resolution image data. The ultrafine coaxial cable 100 may transmit analog signals in other various embodiments.

The coaxial cable 100 includes a center conductor 110, an insulator 120, a cable shield 130, and an outer jacket 140. In an exemplary embodiment, the coaxial cable 100 is manufactured by an additive manufacturing process. For example, the coaxial cable 100 is built up in layers. In an exemplary embodiment, insulator 120 is applied directly to the center conductor 110 by an additive manufacturing process. The cable shield 130 is applied directly to the insulator 120 by an additive manufacturing process. The outer jacket 140 is applied directly to the cable shield 130 by an additive manufacturing process.

In an exemplary embodiment, the center conductor 110 includes a solid core wire 112. For example, the wire 112 may be a copper wire or a copper alloy wire. In various embodiments, the wire 112 may be a silver plated copper alloy wire. In various embodiments, the center conductor 110 may be a small diameter wire. For example, the center conductor 110 may have a diameter of approximately 0.0005 inch (0.5 mils) (0.0127 mm). The center conductor 110 may be stranded wire in alternative embodiments.

In an exemplary embodiment, the insulator 120 is formed from a dielectric coating 122 applied to the center conductor 110. The dielectric coating 122 may be manufactured from a polymer, such as a polyethylene. In other various embodiments, the dielectric coating 122 may be manufactured from a fluoropolymer, such as polytetrafluoroethylene (PTFE). Other types of polymers may be used in alternative embodiments, such as a polyimide. In an exemplary embodiment, the insulator 120 has a relatively small outer diameter. For example, the outer diameter may be smaller than conventional insulators manufactured from an extrusion process, which have extrusion limits of approximately 0.0008 inch (0.020 mm) for typical extrusion materials. In an exemplary embodiment, the insulator 120 has an outer diameter less than 0.002 inch (2.0 mil) (0.051 mm). In an exemplary embodiment, the insulator 120 has a thickness less than 0.001 inch (1.0 mil) (0.0254 mm). In various embodiments, the insulator 120 has a thickness less than 0.00075 inch (0.75 mil) (0.0191 mm).

In various embodiments, the dielectric coating 122 is applied to the center conductor 110 by a dip coating process. For example, the center conductor 110 may be dip coated in a polymer bath to apply the dielectric coating 122 to the center conductor 110. In an exemplary embodiment, the dielectric coating 122 may be cured after the dip coating process. In various embodiments, the dielectric coating 122 may be applied in multiple layers that are dip coated and cured prior to application of the next layer. The number of layers and/or the layer thickness of the dielectric coating 122 is selected to control the thickness of the insulator 120, such as to control electrical characteristics of the coaxial cable 100 by controlling spacing between the center conductor 110 and the cable shield 130. For example, the thickness of the insulator 120 may be selected to achieve 50 ohms. The thickness of the insulator 120 may correspond to the material of the dielectric coating 122, such as the dielectric constant of the material of the dielectric coating 122. In various embodiments, the dielectric coating 122 may have a low dielectric constant to allow the insulator 120 to have a reduced thickness. For example, the dielectric coating 122 may have a dielectric constant of approximately 2.0. In various embodiments, the dielectric coating 122 may be manufactured from a PTFE material having a dielectric constant of approximately 2.1. Other materials may be used in alternative embodiments having a higher or lower dielectric constant than the PTFE material.

In various embodiments, the dielectric coating 122 is formed to provide the insulator 120 with a microcellular structure having air pockets. For example, the dielectric coating 122 undergoes a foaming process in which air bubbles are introduced into the dielectric coating 122. In various embodiments, the dielectric coating 122 passes through a CO₂ bath where bubbles are introduced into the dielectric coating 122. Other foaming processes may be used in alternative embodiments. Optionally, the foaming process may occur prior to the curing process. The foaming process may lower the dielectric constant of the insulator 120. For example, the foaming process may lower the dielectric constant by approximately 25%. In various embodiments, the dielectric coating 122 is manufactured from a polyimide material that is foamed to introduce air pockets in the polyimide material achieving a dielectric constant of approximately 1.4. The dielectric constant of the foamed material may be increased or decreased depending on the polymer material of the dielectric coating 122 and the amount of foaming, which increases or decreases the volume of air bubbles in the dielectric coating 122. The lower dielectric constant of the foamed material allows the insulator 120 to have a reduced thickness, thus decreasing the overall diameter of the coaxial cable 100.

In various embodiments, the dielectric coating 122 is formed to provide the insulator 120 with an aerogel structure having air pockets. The aerogel structure may have between 50% and 99.98% air by volume. In various embodiments, the aerogel structure may have greater than 90% porosity. For example, the dielectric coating 122 undergoes a gelling process and then a drying process to provide the aerogel structure. The gelling process includes applying the dielectric coating 122 to the center conductor 110 as a gel. During the drying process of the aerogel, liquid is removed and replaced with air, while keeping the aerogel intact. The aerogel may be freeze-dried. The aerogel process lowers the dielectric constant of the insulator 120. For example, the aerogel process may lower the dielectric constant by approximately 50%. In various embodiments, the dielectric coating 122 is manufactured from an aerogel material having a dielectric constant of below 1.5. The dielectric constant of the aerogel material may be approximately 1.1 in various embodiments. The lower dielectric constant of the foamed material allows the insulator 120 to have a reduced thickness, thus decreasing the overall diameter of the coaxial cable 100.

In various embodiments, the outer surface of the insulator 120 undergoes an etching process prior to forming the cable shield 130 on the insulator 120. The etching process improves bonding between the cable shield 130 and the insulator 120. In an exemplary embodiment, the insulator 120 may undergo a chemical etching process. In other various embodiments, the insulator 120 may undergo a plasma etching process.

In an exemplary embodiment, the cable shield 130 is formed from a metallization layer 132 applied to the outer surface of the insulator 120. The metallization layer 132 may be applied directly to the outer surface of the insulator 120. The metallization layer 132 may be a copper layer or a copper alloy layer plated to the insulator 120, the metallization layer 132 is a silver plated copper silver plated copper alloy layer. Optionally, multiple metallization layers 132 may be applied to the insulator 120. In an exemplary embodiment, the metallization layer 132 is a plating layer applied by a plating process. For example, the metallization layer 132 may be an electroless plating layer. The metallization layer 132 may be applied by dip coating the metallization layer 132 onto the insulator 120. In other various embodiments, the metallization layer 132 is applied by a vapor deposition process. In alternative embodiments, the metallization layer 132 is an electrolytic plating layer. For example, the metallization layer 132 is applied by an electroplating process. In various embodiments, the metallization layer 132 may include conductive ink layer applied to the outer surface of the insulator 120 and one or more electroplating layers added to the conductive ink layer. The plating process is controlled to control a thickness of the metallization layer 132 on the insulator 120, such as to control impedance and/or capacitance and/or other electrical characteristics of the cable shield 130. The cable shield 130 provides electrical shielding for the center conductor 110. In an exemplary embodiment, the additive process of applying the metallization layer 132 to the insulator 120 provides a relatively thin cable shield 130. In various embodiments, the thickness of the cable shield 130 may be less than 0.0005 inch (0.5 mil) (0.0127 mm). In an exemplary embodiment, the thickness of the cable shield 130 may be less than 0.0003 inch (0.3 mil) (0.00762 mm). The additive manufactured plating process allows the cable shield 130 to be very thin, particularly compared to a serve or wrapped wire shield of conventional coaxial cables, thus decreasing the overall diameter of the coaxial cable 100.

In an exemplary embodiment, the outer jacket 140 is formed from a dielectric coating 142 applied to the outer surface of the cable shield 130. In various embodiments, the dielectric coating 142 is applied to the cable shield 130 by a dip coating process. For example, the coaxial cable 100 may be dip coated in a polymer bath to apply the dielectric coating 142 to the cable shield 130. In an exemplary embodiment, the dielectric coating 142 may be cured after the dip coating process. In various embodiments, the dielectric coating 142 may be applied in multiple layers that are dip coated and cured prior to application of the next layer. The number of layers in the dielectric coating 122 is selected to control the thickness of the outer jacket 140. The additive manufactured dip coating process of applying the outer jacket 140 to the cable shield 130 allows the outer jacket 140 to be relatively thin, particularly compared to a taped mylar jacket of conventional coaxial cables, thus decreasing the overall diameter of the coaxial cable 100. In various embodiments, the thickness of the outer jacket 140 may be less than 0.0005 inch (0.5 mil) (0.0127 mm). In an exemplary embodiment, the thickness of the outer jacket 140 may be less than 0.0003 inch (0.3 mil) (0.00762 mm).

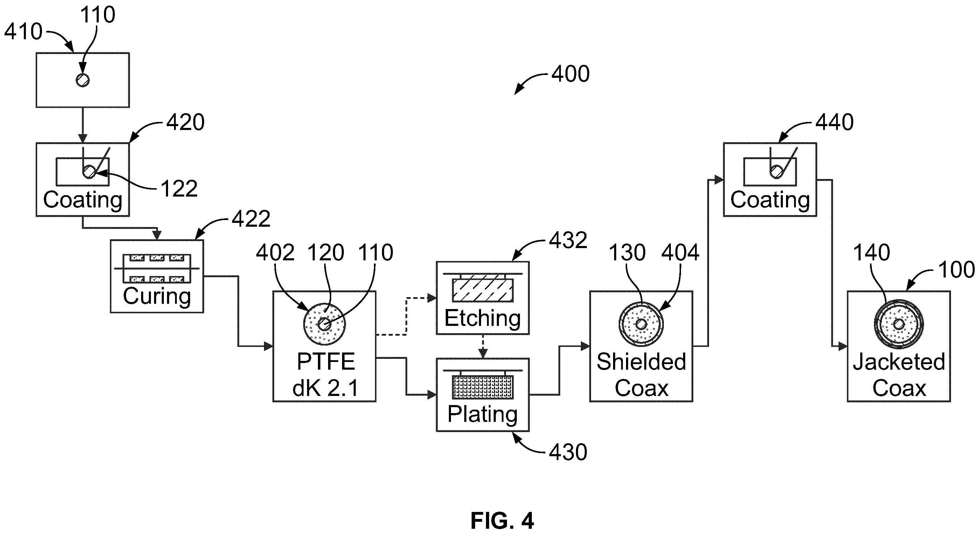

Figure 4 is a flow chart 400 showing an exemplary method of manufacturing a coaxial cable. The method includes providing 410 a center conductor 110. The center conductor 110 may be provided as a continuous center conductor, such as on a spool for making various length coaxial cables. Alternatively, the center conductor 110 may be provided as a discrete center conductor having a predetermined length.

The method includes coating 420 the center conductor 110 with a dielectric coating 122 to form an insulator 120 surrounding the center conductor 110. The dielectric coating 122 is applied directly to the center conductor 110. The dielectric coating 122 may be manufactured from a polymer material. For example, the dielectric coating may be a fluoropolymer, such as polytetrafluoroethylene (PTFE). Other types of polymers may be used in alternative embodiments, such as a polyimide. In various embodiments, the coating process is a dip coating process. For example, the center conductor 110 may be dip coated in a polymer bath to apply the dielectric coating 122 to the center conductor 110.

In an exemplary embodiment, the method includes curing 422 the dielectric coating 122 after the dip coating process. The curing may be performed by heaters. For example, the dip coated structure may be passed through an oven or other heating device. In various embodiments, the dielectric coating 122 may be applied in multiple layers that are dip coated and cured prior to application of the next layer. The additive process of applying the dielectric coating 122 to the center conductor 110 provides a thin insulator 120, particularly compared to conventional coaxial cables having extruded insulators, which have extrusion limits of approximately 0.0008 inch (0.020 mm) for typical extrusion materials. The reduction in thickness of the insulator 120 decreases the overall diameter of the coaxial cable 100. After the dip coating (and curing) process, an insulated conductor core 402 is provided. The insulated conductor core may be further processed to form the coaxial cable. The insulator conductor core may be wound on a reel for processing at a later time. Alternatively, the insulated conductor core may be further processed by immediate transfer to another processing machine.

In an exemplary embodiment, the method includes the step of covering 430 the insulator 120 with a metallization layer to form a cable shield 130 on the outer surface of the insulator 120. After the metallization layer is applied, a shielded conductor core 404 is provided. The metallization layer may be applied directly to the outer surface of the insulator 120. Optionally, multiple metallization layers may be applied to the insulator 120. In an exemplary embodiment, the covering process is a plating process, such as an electroless plating process. For example, the metallization layer may be applied by dip coating the metallization layer onto the insulator 120. In other various embodiments, the plating process may be a vapor deposition process. In alternative embodiments, the plating process is an electrolytic plating process. For example, the metallization layer may be applied by an electroplating process on a conductive ink layer that is applied to the outer surface of the insulator 120. One or more electroplating layers may be added to the conductive ink layer. The additive process of applying the metallization layer to the insulator 120 provides a relatively thin cable shield 130, particularly compared to conventional coaxial cables having wrapped wires wrapped around the insulator. The reduction in thickness of the cable shield 130 decreases the overall diameter of the coaxial cable 100.

In various embodiments, the method may include an optional step of etching 432 the outer surface of the insulator 120 prior to forming a cable shield 130 on the insulator 120. The etching process may improve bonding between the cable shield 130 and the insulator 120. In an exemplary embodiment, the insulator 120 may undergo a vapor etching process. In other various embodiments, the insulator 120 may undergo a plasma etching process.

In an exemplary embodiment, the method includes the step of covering 440 the cable shield 130 with an outer jacket 140. The outer jacket 140 is formed from a dielectric coating applied to the outer surface of the cable shield 130. In various embodiments, the covering process may be a dip coating process. For example, the shielded conductor core 404 may be dip coated in a polymer bath to apply the dielectric coating to the cable shield 130. In an exemplary embodiment, the method may include the step of curing the dielectric coating after the dip coating process. In various embodiments, the dielectric coating may be applied in multiple layers that are dip coated and cured. The additive manufactured dip coating process of applying the outer jacket 140 to the cable shield 130 provides a thin outer jacket 140, particularly compared to conventional coaxial cables that have taped mylar jacket applied to the outer layer, thus decreasing the overall diameter of the coaxial cable 100.

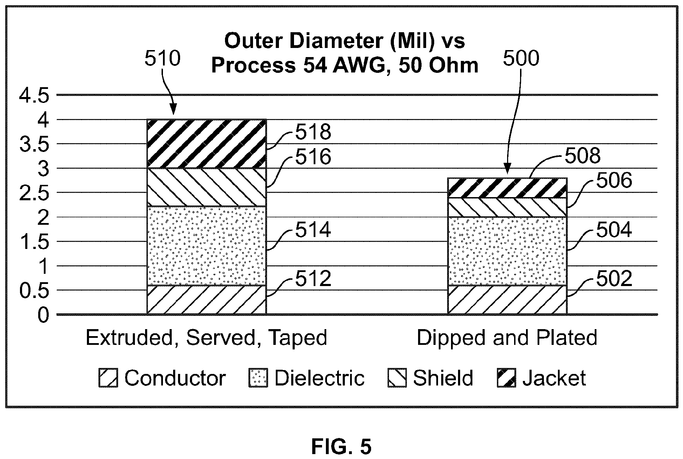

Figure 5 is a chart showing cable outer diameter versus manufacturing process for a coaxial cable 500 manufactured according to the method shown in Figure 4 (for example, additive manufactured by dip coating and plating) versus a conventional coaxial cable 510 manufactured according to conventional manufacturing methods (for example, extruded insulator, served and taped). The coaxial cable 500 includes a center conductor 502, an insulator 504, a cable shield 506, and an outer jacket 508. The conventional coaxial cable 510 includes a center conductor 512, an insulator 514, a cable shield 516, and an outer jacket 518. The chart shows the total outer diameter of each of the cables 500, 510. The chart shows the total thickness of each layer (for example, total thickness is twice of the layer thickness because each layer is split on opposite sides of a central plane through the total diameter of the coaxial cable) for each of the cables 500, 510. In an exemplary embodiment, the coaxial cable 500 has a smaller outer diameter compared to the conventional coaxial cable 510. In an exemplary embodiment, one or more layers of the coaxial cable 500 are thinner than corresponding layers of the conventional coaxial cable 510, such as the insulator and/or the cable shield and/or the outer jacket.

In an exemplary embodiment, the coaxial cable 500 has an outer diameter of approximately 0.0027 inch (2.7 mil) (0.0686 mm). The center conductor 502 has a diameter of approximately 0.0005 inch (0.5 mil) (0.0127 mm). The insulator 504 has a diameter of approximately 0.002 inch (2.0 mil) (0.051 mm), a total thickness of approximately 0.0015 inch (1.5 mil) (0.0038 mm) and thus having a layer thickness of 0.00075 inch (0.75 mil) (0.0191 mm)surrounding the center conductor 502. In an exemplary embodiment, the thickness of the insulator is less than 50% of the total outer diameter of the coaxial cable 500, such as approximately 45% of the total outer diameter of the coaxial cable 500. The cable shield 506 has a diameter of approximately 0.0024 inch (2.4 mil) (0.061 mm), a total thickness of approximately 0.0004 inch (0.4 mil) (0.010 mm), thus having a layer thickness of 0.0002 inch (0.2 mil) (0.0051 mm) surrounding the insulator 504. In an exemplary embodiment, the thickness of the cable shield 506 is less than 20% of the total outer diameter of the coaxial cable 500, such as approximately 10% the total outer diameter of the coaxial cable 500. The outer jacket 508 has a diameter of approximately 0.0027 inch (2.7 mil) (0.0686 mm), a total thickness of approximately 0.0003 inch (0.3 mil) (0.00762 mm), thus having a layer thickness of 0.15 mil surrounding the cable shield 506. In an exemplary embodiment, the thickness of the outer jacket 508 is less than 20% of the total outer diameter of the coaxial cable 500, such as approximately 10% the total outer diameter of the coaxial cable 500.

In an exemplary embodiment, the conventional coaxial cable 510 has an outer diameter of approximately 0.004 inch (4.0 mil) (0.101 mm). The conventional center conductor 512 has a diameter of approximately 0.0005 inch (0.5 mils) (0.0127 mm) mil. The conventional insulator 514 has a diameter of approximately 0.0022 inch (2.2 mil) (0.056 mm), a total thickness of approximately 0.0017 inch (1.7 mil) (0.043 mm) and thus having a layer thickness of 0.00085 inch (0.85 mil) (0.022 mm) surrounding the center conductor 512. In an exemplary embodiment, the thickness of the insulator is greater than 50% of the total outer diameter of the conventional coaxial cable 510, such as approximately 55% of the total outer diameter of the conventional coaxial cable 510. The conventional cable shield 516 has a diameter of approximately 0.003 inch (3.0 mil) (0.076 mm), a total thickness of approximately 0.0008 inch (0.8 mil) (0.020 mm), thus having a layer thickness of 0.0004 inch (0.4 mil) (0.010 mm) surrounding the conventional insulator 514. In an exemplary embodiment, the thickness of the conventional cable shield 516 is greater than 20% of the total outer diameter of the conventional coaxial cable 510. The conventional outer jacket 518 has a diameter of approximately 0.004 inch (4.0 mil) (0.101 mm), a total thickness of approximately 0.001 inch (1.0 mil) (0.0254 mm), thus having a layer thickness of 0.0005 inch (0.5 mils) (0.0127 mm)surrounding the conventional cable shield 516. In an exemplary embodiment, the thickness of the conventional outer jacket 518 is greater than 20% of the total outer diameter of the conventional coaxial cable 510, such as greater than 25%.

In an exemplary embodiment, the coaxial cable 500 has a smaller diameter than the conventional coaxial cable 510. For example, the coaxial cable 500 may have a diameter that is reduced by at least 25% compared to the conventional coaxial cable 510. The insulator 504 may have a thickness that is thinner than the conventional insulator 514. For example, the insulator 504 may have a thickness that is reduced by at least 5% compared to the conventional insulator 514. The cable shield 506 may have a thickness that is thinner than the conventional cable shield 516. For example, the cable shield 506 may have a thickness that is reduced by approximately 50% compared to the conventional cable shield 516. The outer jacket 508 may have a thickness that is thinner than the conventional outer jacket 518. For example, the outer jacket 508 may have a thickness that is reduced by approximately 50% compared to the conventional outer jacket 518.

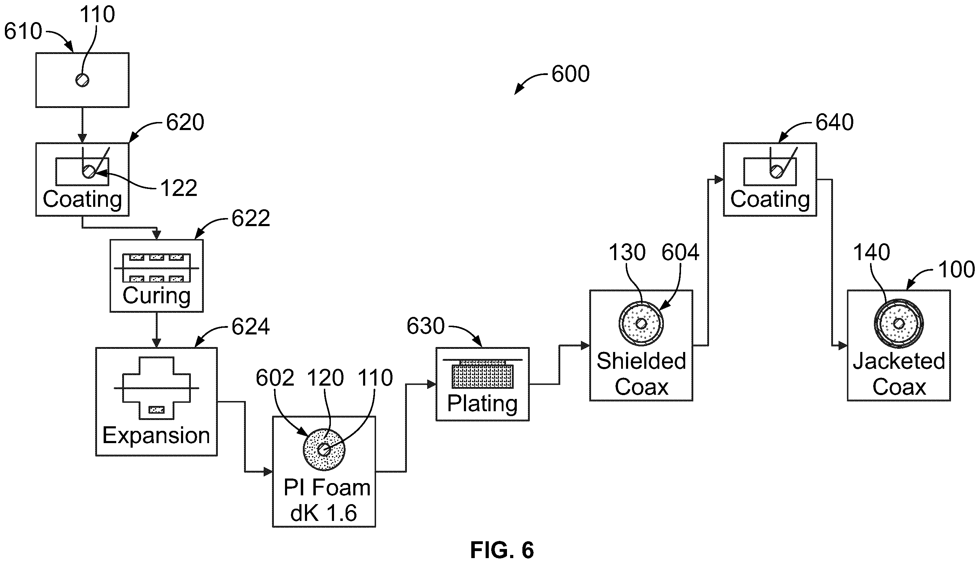

Figure 6 is a flow chart 600 showing an exemplary method of manufacturing a coaxial cable. The method includes providing 610 a center conductor 110. The center conductor 110 may be provided as a continuous center conductor, such as on a spool for making various length coaxial cables. Alternatively, the center conductor 110 may be provided as a discrete center conductor having a predetermined length.

The method includes coating 620 the center conductor 110 with a dielectric coating 122 to form an insulator 120 surrounding the center conductor 110. The dielectric coating 122 is applied directly to the center conductor 110. The dielectric coating 122 may be manufactured from a polymer material. For example, the dielectric coating may be a polyimide. In various embodiments, the coating process is a dip coating process. For example, the center conductor 110 may be dip coated in a polymer bath to apply the dielectric coating 122 to the center conductor 110. In an exemplary embodiment, the method includes curing 622 the dielectric coating 122 after the dip coating process. In an exemplary embodiment, the method includes expanding 624 the dielectric coating 122. For example, the expansion of the dielectric coating may include the step of foaming the dielectric coating. Air bubbles may be added to the dielectric coating. The foaming process forms an insulator 120 having a microcellular structure, such as a structure having air pockets. The foaming process lowers the dielectric constant of the insulator 120. For example, the insulator 120 may have a dielectric constant of approximately 1.6. The additive process of applying the dielectric coating 122 to the center conductor 110 provides a thin insulator 120, particularly compared to conventional coaxial cables having extruded insulators. The reduction in thickness of the insulator 120 decreases the overall diameter of the coaxial cable 100. After the dip coating (and curing) process, an insulated conductor core 602 is provided.

In an exemplary embodiment, the method includes the step of plating 630 the insulator 120 with a metallization layer to form a cable shield 130 on the outer surface of the insulator 120. After the metallization layer is applied, a shielded conductor core 604 is provided. The metallization layer may be applied directly to the outer surface of the insulator 120. Optionally, multiple metallization layers may be applied to the insulator 120. In an exemplary embodiment, the plating process is an electroless plating process. For example, the metallization layer may be applied by dip coating the metallization layer onto the insulator 120. In other various embodiments, the plating process may be a vapor deposition process. In alternative embodiments, the plating process is an electrolytic plating process. For example, the metallization layer may be applied by an electroplating process on a conductive ink layer that is applied to the outer surface of the insulator 120. One or more electroplating layers may be added to the conductive ink layer. In various embodiments, the method may include an optional step of etching the outer surface of the insulator 120 prior to forming a cable shield 130 on the insulator 120. The additive process of applying the metallization layer to the insulator 120 provides a relatively thin cable shield 130, particularly compared to conventional coaxial cables having wrapped wires wrapped around the insulator. The reduction in thickness of the cable shield 130 decreases the overall diameter of the coaxial cable 100.

In an exemplary embodiment, the method includes the step of covering 640 the cable shield 130 with an outer jacket 140. The outer jacket 140 is formed from a dielectric coating applied to the outer surface of the cable shield 130. In various embodiments, the covering process may be a dip coating process. For example, the shielded conductor core 604 may be dip coated in a polymer bath to apply the dielectric coating to the cable shield 130. In an exemplary embodiment, the method may include the step of curing the dielectric coating after the dip coating process. In various embodiments, the dielectric coating may be applied in multiple layers that are dip coated and cured. The additive manufactured dip coating process of applying the outer jacket 140 to the cable shield 130 provides a thin outer jacket 140, particularly compared to conventional coaxial cables that have taped mylar jacket applied to the outer layer, thus decreasing the overall diameter of the coaxial cable 100.

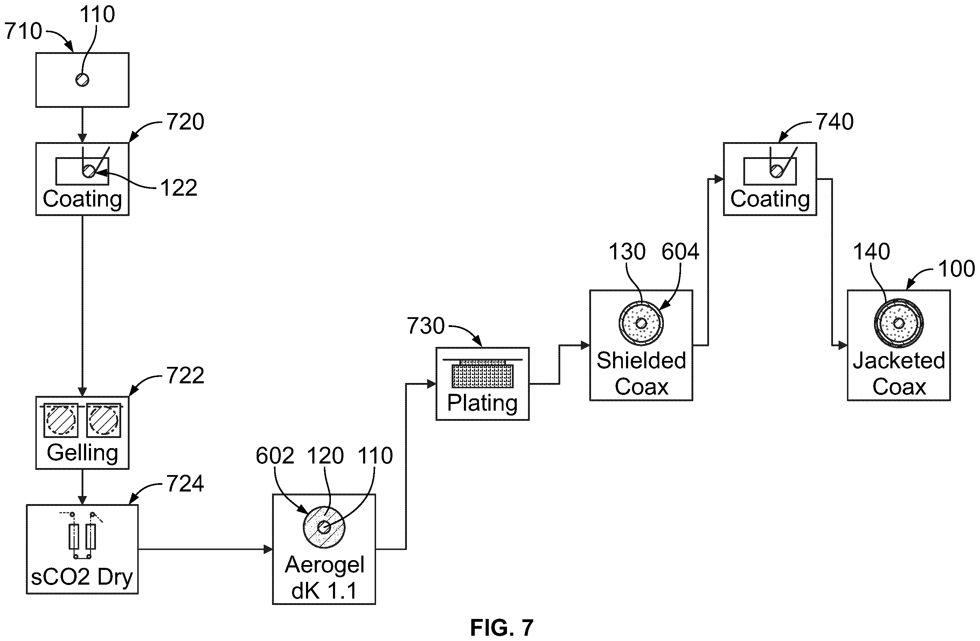

Figure 7 is a flow chart 700 showing an exemplary method of manufacturing a coaxial cable. The method includes providing 710 a center conductor 110. The center conductor 110 may be provided as a continuous center conductor, such as on a spool for making various length coaxial cables. Alternatively, the center conductor 110 may be provided as a discrete center conductor having a predetermined length.

The method includes coating 720 the center conductor 110 with a dielectric coating 122 to form an insulator 120 surrounding the center conductor 110. In an exemplary embodiment, the insulator 120 is an aerogel having a low dielectric constant. The dielectric coating 122 is applied directly to the center conductor 110. The dielectric coating 122 may be manufactured from a polymer material. In various embodiments, the coating process is a dip coating process. For example, the center conductor 110 may be dip coated in a polymer bath to apply the dielectric coating 122 to the center conductor 110. The dip coating may be a sol-gel material. In an exemplary embodiment, the method includes the step of gelling 722 the dielectric coating 122. For example, the method involves the conversion of the liquid system (sol) into a gel phase through chemical reactions. The method includes the step of drying 724 the dielectric coating material. The drying step may include extracting the liquid component of the gel through supercritical drying or freeze-drying to allow the liquid to be slowly dried off without causing the solid matrix in the gel to collapse, such as from capillary action. For example, the solvent will be removed from the gel structure, followed by filling the network with air, to form the aerogel structure. The dielectric coating may include silica, alumina, and/or carbon material to form the gel. The dielectric coating may be various polymers that are cross-linked in solution. The forming process of the aerogel lowers the dielectric constant of the insulator 120. For example, the insulator 120 may have a dielectric constant of approximately 1.1. The additive process of applying the dielectric coating 122 to the center conductor 110 provides a thin insulator 120, particularly compared to conventional coaxial cables having extruded insulators. The reduction in thickness of the insulator 120 decreases the overall diameter of the coaxial cable 100. After the aerogel process, an insulated conductor core 702 is provided.

In an exemplary embodiment, the method includes the step of plating 730 the insulator 120 with a metallization layer to form a cable shield 130 on the outer surface of the insulator 120. After the metallization layer is applied, a shielded conductor core 704 is provided. The metallization layer may be applied directly to the outer surface of the insulator 120. Optionally, multiple metallization layers may be applied to the insulator 120. In an exemplary embodiment, the plating process is an electroless plating process. For example, the metallization layer may be applied by dip coating the metallization layer onto the insulator 120. In other various embodiments, the plating process may be a vapor deposition process. In alternative embodiments, the plating process is an electrolytic plating process. For example, the metallization layer may be applied by an electroplating process on a conductive ink layer that is applied to the outer surface of the insulator 120. One or more electroplating layers may be added to the conductive ink layer. In various embodiments, the method may include an optional step of etching the outer surface of the insulator 120 prior to forming a cable shield 130 on the insulator 120. The additive process of applying the metallization layer to the insulator 120 provides a relatively thin cable shield 130, particularly compared to conventional coaxial cables having wrapped wires wrapped around the insulator. The reduction in thickness of the cable shield 130 decreases the overall diameter of the coaxial cable 100.

In an exemplary embodiment, the method includes the step of covering 740 the cable shield 130 with an outer jacket 140. The outer jacket 140 is formed from a dielectric coating applied to the outer surface of the cable shield 130. In various embodiments, the covering process may be a dip coating process. For example, the shielded conductor core 704 may be dip coated in a polymer bath to apply the dielectric coating to the cable shield 130. In an exemplary embodiment, the method may include the step of curing the dielectric coating after the dip coating process. In various embodiments, the dielectric coating may be applied in multiple layers that are dip coated and cured. The additive manufactured dip coating process of applying the outer jacket 140 to the cable shield 130 provides a thin outer jacket 140, particularly compared to conventional coaxial cables that have taped mylar jacket applied to the outer layer, thus decreasing the overall diameter of the coaxial cable 100.

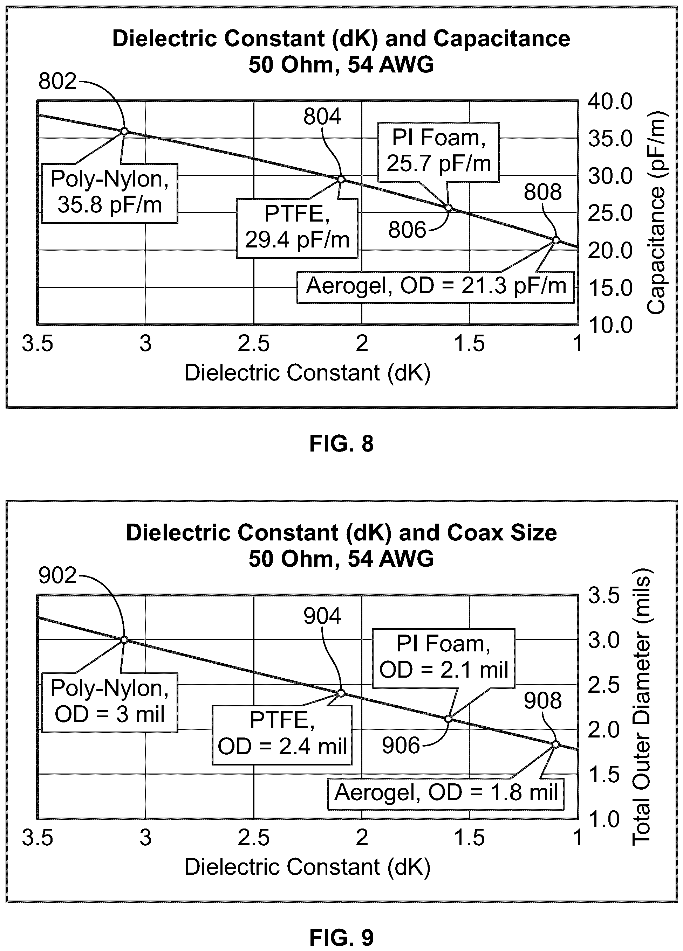

Figure 8 is a chart showing the dielectric constant and the capacitance for a 50 Ohm, 54 AWG coaxial cable using various insulator materials. In the illustrated embodiment, a first insulator 802 is shown with a conventional extruded insulator; a second insulator 804 is shown manufactured from a PTFE according to the method described in Figure 4; a third insulator 806 is shown manufactured from a polyimide foam according to the method described in Figure 6; and a fourth insulator 808 is shown manufactured from an aerogel according to the method described in Figure 7. In the illustrated embodiment, the first insulator 802 has a dielectric constant of approximately 3.1 and a capacitance of 35.8 pF/m; the second insulator 804 has a dielectric constant of approximately 2.1 and a capacitance of 29.4 pF/m; the third insulator 806 has a dielectric constant of approximately 1.6 and a capacitance of 25.7 pF/m; and the fourth insulator 808 has a dielectric constant of approximately 1.1 and a capacitance of 21.3 pF/m. The lower capacitance of the insulators 804, 806, 808 manufactured from the additive manufacturing processes described in the methods of Figures 4, 6, and 7, respectively, is greatly reduced compared to a conventional extruded, served and taped coaxial cable providing ultrafine coaxial cables having improved electrical characteristics for use in certain applications, such as medical or surgical applications.

Figure 9 is a chart showing the dielectric constant and the outer diameter for a 50 Ohm, 54 AWG coaxial cable using various insulator materials. In the illustrated embodiment, a first insulator 902 is shown with a conventional extruded insulator; a second insulator 904 is shown manufactured from a PTFE according to the method described in Figure 4; a third insulator 906 is shown manufactured from a polyimide foam according to the method described in Figure 6; and a fourth insulator 908 is shown manufactured from an aerogel according to the method described in Figure 7. In the illustrated embodiment, the first insulator 902 has a dielectric constant of approximately 3.1 and an outer diameter of 0.003 inch (3.0 mil) (0.076 mm); the second insulator 904 has a dielectric constant of approximately 2.1 and an outer diameter of 0.0024 inch (2.4 mil) (0.061 mm); the third insulator 906 has a dielectric constant of approximately 1.6 and an outer diameter of 0.0021 inch (2.1 mil) (0.053 mm); and the fourth insulator 908 has a dielectric constant of approximately 1.1 and an outer diameter of 0.0018 inch (1.8 mil) (0.046 mm). The outer diameter of the insulators 904, 906, 908 manufactured from the additive manufacturing processes described in the methods of Figures 4, 6, and 7, respectively, is greatly reduced compared to a conventional extruded, served and taped coaxial cable providing ultrafine coaxial cables for use in certain applications, such as medical or surgical applications.

It is to be understood that the above description is intended to be illustrative, and not restrictive. For example, the above-described embodiments (and/or aspects thereof) may be used in combination with each other. In addition, many modifications may be made to adapt a particular situation or material to the teachings of the invention without departing from its scope. Dimensions, types of materials, orientations of the various components, and the number and positions of the various components described herein are intended to define parameters of certain embodiments, and are by no means limiting and are merely exemplary embodiments. Many other embodiments and modifications within the scope of the claims will be apparent to those of skill in the art upon reviewing the above description. The scope of the invention should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects. Further, the limitations of the following claims are not written in means - plus-function format and are not intended to be interpreted based on 35 U.S.C. § 112(f), unless and until such claim limitations expressly use the phrase "means for" followed by a statement of function void of further structure.

## Claims

1. A method of manufacturing a coaxial cable (100) comprising:
providing a center conductor (110);
coating (420) the center conductor (110) with a dielectric coating (122) to form an insulator (120) surrounding the center conductor (110);
covering the insulator (120) with a metallization layer (132) to form a cable shield (130) surrounding the insulator (120); and
covering the cable shield (130) with an outer jacket (140); wherein the coaxial cable (100) has a wire gauge of 54 AWG or smaller.

2. The method of claim 1, wherein the coating (420) includes the steps of dip coating and curing (442) the dielectric coating (122) in multiple layers (32).

3. The method of claim 1 or 2, wherein the coating (420) includes the step of foaming the dielectric coating (122) such that the insulator (120) has a microcellular structure with air pockets.

4. The method of claim 1 or 2, wherein the coating (420) includes the steps of gelling (722) the dielectric coating (122) and drying the dielectric coating (122) such that the insulator (120) is an aerogel structure.

5. The method of any preceding claim, further comprising etching an outer surface of the insulator (120), the metallization layer (132) being plated to the etched outer surface.

6. The method of any preceding claim, wherein the covering the insulator (120) with the metallization layer (132) includes electroless plating or electrolytic plating or vapor depositing the metallization layer (132) onto the insulator (120).

7. The method of any preceding claim, wherein the covering of the cable shield (130) with the outer jacket (140) includes the steps of dip coating and curing (442) a dielectric coating (122) onto the metallization layer (132) to form the cover.

8. The method of any preceding claim, wherein the dielectric coating (122) is applied to the center conductor (110) by a process other than extrusion.

9. A coaxial cable (100) comprising:
a center conductor (110);
a dielectric coating (122) applied to the center conductor (110), the dielectric coating (122) forming an insulator (120) surrounding the center conductor (110);
a metallization layer (132) applied to the insulator (120), the metallization layer (132) forming a cable shield (130) surrounding the insulator (120); and
an outer jacket (140) covering the cable shield (130); wherein the coaxial cable (100) has a wire gauge of 54 AWG or smaller.

10. The coaxial cable of claim 9, wherein the dielectric coating (122) comprises one of a polytetrafluoroethylene (PTFE) material, a polyethylene material, a polyimide foam, or a polyethylene foam.

11. The coaxial cable of claim 9, wherein the dielectric coating (122) comprises a polymer-based aerogel or a microcellular structure having air pockets.

12. The coaxial cable of claim 9, 10 or 11, wherein the outer jacket (140), the dielectric coating (122), or both the outer jacket (140) and the dielectric coating (122) are dip coated and cured in one or more layers.

13. The coaxial cable of any one of claims 9 to 12, wherein the dielectric coating (122) has an outer diameter less than 0.002 inch (0.051 mm), the metallization layer (132) has an outer diameter less than 0.0024 inch (0.0635 mm), and the outer jacket (140) has an outer diameter less than 0.003 inch (0.076 mm).

14. The coaxial cable of any one of claims 9 to 13, wherein the metallization layer (132) has a thickness less than 20% of a total outer diameter of the coaxial cable (100) and the outer jacket (140) has a thickness less than 20% of the total coaxial cable (100).

15. A medical device (10) comprising:
an instrument (20) performing an operation; and
an ultrafine coaxial cable (100) electrically connected to the instrument (10) to transmit data between the instrument (20) and an electrical device (30), the ultrafine coaxial cable (100) comprising a coaxial cable as claimed in any one of claims 9 to 14.

16. The medical device of claim 15, wherein the instrument (20) is (a) an ultrasound transducer, the ultrafine coaxial cable (100) electrically coupled to the ultrasound transducer, or (b) a camera, the ultrafine coaxial cable (100) electrically coupled to the camera, preferably wherein the medical device (10) further comprises a catheter having a lumen, the ultrafine coaxial cable (100) routed through the lumen of the catheter.
